(19) Europäisches Patentamt
European Patent Office
Office européen des brevets

(11) Veröffentlichungsnummer: **0 358 018**
**A2**

(12) # EUROPÄISCHE PATENTANMELDUNG

(21) Anmeldenummer: 89115320.7

(22) Anmeldetag: 19.08.89

(51) Int. Cl.5: C07D 251/16 , C07D 239/52 , //C07D251/46,C07D251/52, C07D239/47,C07D239/42

(30) Priorität: 03.09.88 DE 3829957

(43) Veröffentlichungstag der Anmeldung: 14.03.90 Patentblatt 90/11

(84) Benannte Vertragsstaaten: BE CH DE FR GB IT LI NL

(71) Anmelder: **BAYER AG**
D-5090 Leverkusen 1 Bayerwerk(DE)

(72) Erfinder: **Riebel, Hans-Jochem, Dr.**
In der Beek 92
D-5600 Wuppertal 1(DE)
Erfinder: **Müller, Klaus-Helmut, Dr.**
Bockhackstrasse 55
D-4000 Düsseldorf 13(DE)

(54) **Verfahren zur Herstellung von Oxyguanidinen.**

(57) Oxyguanidine der allgemeinen Formel (I)

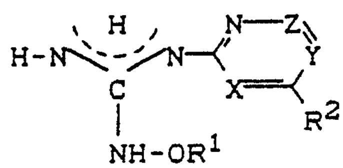

(I)

in welcher
$R^1$ für einen jeweils gegebenenfalls substituierten Rest aus der Reihe Alkyl, Alkenyl, Alkinyl, Cycloalkyl, Cycloalkylalkyl, Aralkyl und Aryl steht,
$R^2$ für Wasserstoff, Halogen, Hydroxy, Amino oder einen gegebenenfalls substituierten Rest aus der Reihe Alkyl, Alkoxy, Alkylthio, Alkylamino und Dialkylamino steht,
X für Stickstoff oder eine CH-Gruppierung steht,
Y für Stickstoff oder eine $CR^3$-Gruppierung steht und
Z für Stickstoff oder eine $CR^4$-Gruppierung steht.
(wobei $R^3$ und $R^4$ die in der Beschreibung angegebenen Bedeutungen haben),
welche als Zwischenprodukte zur Herstellung von Herbiziden verwendet werden können, lassen sich auf einfache Weise in hohen Ausbeuten herstellen, indem man Isothioharnstoffe der allgemeinen Formel (II)

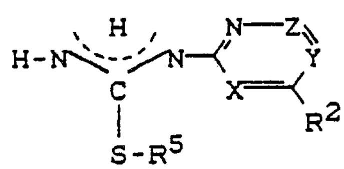

(II)

- oder deren Hydrogenhalogenide -



Xerox Copy Centre

mit Hydroxylaminderivaten der allgemeinen Formel (III)

$H_2N - O - R^1$ (III)

gegebenenfalls in Gegenwart eines Verdünnungsmittels bei Temperaturen zwischen 0 °C und 150 °C umsetzt. Eine definierte Gruppe (II a) von Isothioharnstoffen ist neu; diese neuen Stoffe und ein Verfahren zu ihrer Herstellung sind ebenfalls Gegenstand der Erfindung.

# Verfahren zur Herstellung von Oxyguanidinen

Die Erfindung betrifft ein neues Verfahren und neue Zwischenprodukte zur Herstellung von bekannten Oxyguanidinen

Es ist bekannt, daß man Oxyguanidine erhält, wenn man Cyanaminoverbindungen mit Hydroxylaminderivaten umsetzt (vgl. J. Chem. Soc. C 1966, 2031 - 2038; EP-A 121 082/US-P 4 602 938). Die Herstellung der hierfür als Ausgangsstoffe benötigten Cyanaminoverbindungen ist jedoch in manchen Fällen, insbesondere im Fall von methyl-substituierten Cyanaminotriazinen, welche als Zwischenprodukte für Herbizide von Interesse sind, bisher nur nach technisch kaum praktikablen Methoden möglich. Daher bestand Bedarf an technisch günstigeren Synthesewegen zu Oxyguanidinen.

Es wurde nun gefunden, daß man Oxyguanidine der allgemeinen Formel (I)

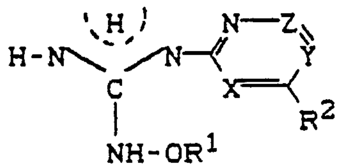

(I)

in welcher

$R^1$ für einen jeweils gegebenenfalls substituierten Rest aus der Reihe Alkyl, Alkenyl, Alkinyl, Cycloalkyl, Cycloalkylalkyl, Aralkyl und Aryl steht,

$R^2$ für Wasserstoff, Halogen, Hydroxy, Amino oder einen gegebenenfalls substituierten Rest aus der Reihe Alkyl, Alkoxy, Alkylthio, Alkylamino und Dialkylamino steht,

X für Stickstoff oder eine CH-Gruppierung steht,

Y für Stickstoff oder eine $CR^3$-Gruppierung steht, worin

$R^3$ für Wasserstoff, Halogen, Cyano, Alkyl, Formyl, Alkylcarbonyl oder Alkoxycarbonyl steht, und

Z für Stickstoff oder eine $CR^4$-Gruppierung steht, worin

$R^4$ für Wasserstoff, Halogen, Hydroxy, Amino oder einen gegebenenfalls substituierten Rest aus der Reihe Alkyl, Alkoxy, Alkylthio, Alkylamino und Dialkylamino steht,

auf einfache Weise in hohen Ausbeuten erhält,

wenn man Isothioharnstoffe der allgemeinen Formel (II)

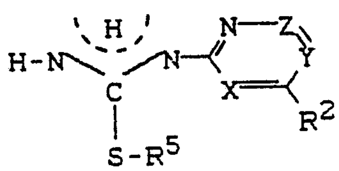

(II)

in welcher

$R^2$, X, Y und Z die oben angegebenen Bedeutungen haben und

$R^5$ für jeweils gegebenenfalls substituiertes Alkyl oder Aralkyl steht,

- oder deren Hydrogenhalogenide -

mit Hydroxylaminderivaten der allgemeinen Formel (III)

$H_2N - O - R^1$ (III)

in welcher

$R^1$ die oben angegebene Bedeutung hat,

gegebenenfalls in Gegenwart eines Verdünnungsmittels bei Temperaturen zwischen 0 °C und 150 °C umsetzt.

Überraschenderweise gelingt es mit Hilfe des erfindungsgemäßen Verfahrens, die Verbindungen der Formel (I) auf relativ einfache Weise in hohen Ausbeuten herzustellen.

Verwendet man beispielsweise N-(4,6-Dimethoxypyrimidin-2-yl)-S-methyl-isothioharnstoff und O-Methylhydroxylamin als Ausgangsstoffe, so kann der Reaktionsablauf durch das folgende Formelschema skizziert werden:

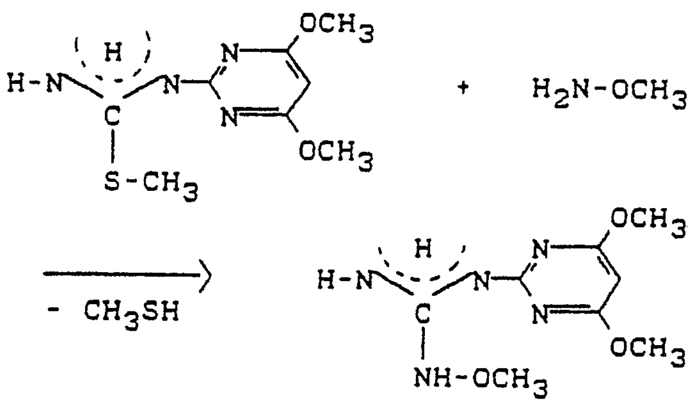


Die als Ausgangsstoffe zu verwendenden Isothioharnstoffe sind durch die Formel (II) allgemein definiert. In Formel (II) stehen vorzugsweise

$R^2$ für Wasserstoff, Fluor, Chlor, Brom, $C_1$-$C_4$-Alkyl, $C_1$-$C_4$-Halogenalkyl, $C_1$-$C_2$-Alkoxy-$C_1$-$C_2$-alkyl, $C_1$-$C_4$-Alkoxy, $C_1$-$C_4$-Halogenalkoxy, $C_1$-$C_4$-Alkylthio, $C_1$-$C_4$-Halogenalkylthio, Amino, $C_1$-$C_4$-Alkylamino, Dimethylamino oder Diethylamino,

X für Stickstoff oder eine CH-Gruppierung,

Y für Stickstoff oder eine $CR^3$-Gruppierung, worin

$R^3$ für Wasserstoff, Fluor, Chlor, Brom, Cyano, Methyl, Formyl, Acetyl, Methoxycarbonyl oder Ethoxycarbonyl steht,

Z für Stickstoff oder eine $CR^4$-Gruppierung, worin

$R^4$ für Wasserstoff, Fluor, Chlor, Brom, $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio, $C_1$-$C_4$-Alkylamino, Dimethylamino oder Diethylamino steht, und

$R^5$ für $C_1$-$C_4$-Alkyl (welches gegebenenfalls durch Cyano, Carboxy oder $C_1$-$C_2$-Alkoxy-carbonyl substituiert ist) oder für Benzyl (welches gegebenenfalls durch Halogen und/oder $C_1$-$C_2$-Alkyl substituiert ist).

Als Ausgangsstoffe besonders bevorzugt werden die Verbindungen der Formel (II), in welcher

$R^2$ für Wasserstoff, Fluor, Chlor, Brom, Methyl, Ethyl, Trifluormethyl, Methoxy, Ethoxy, Difluormethoxy, Methylthio, Ethylthio, Amino, Methylamino, Ethylamino, Dimethylamino oder Diethylamino steht,

X für Stickstoff oder eine CH-Gruppierung steht,

Y für Stickstoff oder eine $CR^3$-Gruppierung steht, worin

$R^3$ für Wasserstoff, Fluor, Chlor oder Methyl steht,

Z für Stickstoff oder eine $CR^4$-Gruppierung steht, worin

$R^4$ für Wasserstoff, Fluor, Chlor, Brom, Methyl, Ethyl, Trifluormethyl, Methoxy, Ethoxy, Propoxy, Isopropoxy, Difluormethoxy, Methylthio, Ethylthio, Methylamino, Ethylamino, Dimethylamino oder Diethylamino steht, und

$R^5$ für Methyl, Ethyl, Carboxymethyl, Methoxycarbonylmethyl, Ethoxycarbonylmethyl oder Benzyl steht

Beispiele für die Ausgangsstoffe der Formel (II) sind in der nachstehenden Tabelle 1 aufgeführt.

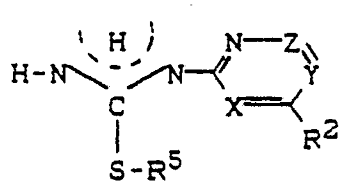

(II)

Tabelle 1: Beispiele für die Ausgangsstoffe der Formel (II)

| $R^5$ | $R^2$ | X | Y | Z |
|---|---|---|---|---|
| $CH_3$ | $CH_3$ | N | CH | $C-CH_3$ |
| $CH_3$ | $CH_3$ | N | CH | $C-OCH_3$ |
| $CH_3$ | $OCH_3$ | N | CH | $C-OCH_3$ |
| $CH_3$ | $OCH_3$ | N | CH | C-Cl |
| $CH_3$ | H | N | CH | $C-CH_3$ |
| $CH_3$ | $CF_3$ | N | CH | $C-OCH_3$ |
| $CH_3$ | $OCH_3$ | N | CH | $C-OCHF_2$ |
| $CH_3$ | $CH_3$ | N | CH | $C-OCHF_2$ |
| $CH_3$ | $CH_3$ | N | N | $C-CH_3$ |
| $CH_3$ | $CH_3$ | N | N | $C-OCH_3$ |
| $CH_3$ | $OCH_3$ | N | N | $C-OCH_3$ |
| $CH_3$ | $C_2H_5$ | N | CH | $C-OCH_3$ |
| $CH_3$ | $CH_3$ | N | N | $C-OC_2H_5$ |
| $CH_3$ | $C_2H_5$ | N | N | $C-OCH_3$ |
| $CH_3$ | $CH_3$ | N | N | C-Cl |
| $CH_3$ | $CH_3$ | CH | N | $C-CH_3$ |
| $CH_3$ | $OCH_3$ | CH | N | $C-OCH_3$ |
| $CH_3$ | $CH_3$ | N | CH | $C-SCH_3$ |
| $CH_3$ | $CH_3$ | N | CH | $C-N(CH_3)_2$ |
| $CH_3$ | $OCH_3$ | N | CH | $C-SCH_3$ |

Tabelle 1 - Fortsetzung

| $R^5$ | $R^2$ | X | Y | Z |
|---|---|---|---|---|
| $CH_3$ | $OCH_3$ | N | N | $C-NHC_2H_5$ |
| $CH_3$ | $OC_2H_5$ | N | N | $C-NHCH_3$ |
| $CH_3$ | $CH_3$ | CH | CH | $C-CH_3$ |
| $CH_3$ | $OCHF_2$ | N | CH | $C-OCHF_2$ |

Die Ausgangsstoffe der Formel (II) sind bekannt und/oder können nach an sich bekannten Verfahren hergestellt werden (vgl. EP-A 117 014, S. 28).

Neu sind mit Ausnahme von N-(4,6-Dimethoxy-s-triazin-2-yl)-S-methyl-isothioharnstoff (vgl. EP-A 117 014, S. 28) die Verbindungen der Formel (IIa)

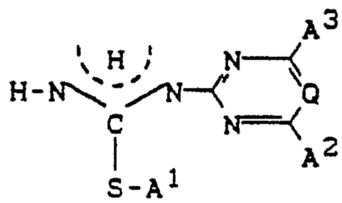

(IIa)

in welcher

$A^1$ für jeweils gegebenenfalls substituiertes Alkyl oder Aralkyl steht,

$A^2$ für Halogen oder einen gegebenenfalls substituierten Rest aus der Reihe Alkyl, Alkoxy, Alkylthio, Alkylamino und Dialkylamino steht,

$A^3$ für Wasserstoff, Halogen oder einen gegebenenfalls substituierten Rest aus der Reihe Alkyl, Alkoxy, Alkylthio, Alkylamino und Dialkylamino steht, und

Q für Stickstoff oder eine CH-Gruppierung steht,

sowie deren Hydrogenhalogenide.

Bevorzugte neue Verbindungen der Formel (IIa) sind diejenigen, bei denen

$A^1$ für $C_1$-$C_4$-Alkyl (welches gegebenenfalls durch Cyano, Carboxy oder $C_1$-$C_4$-Alkoxy-carbonyl substituiert ist) oder für Benzyl (welches gegebenenfalls durch Halogen und/oder $C_1$-$C_2$-Alkyl substituiert ist) steht,

$A^2$ für Fluor, Chlor, Brom, $C_1$-$C_4$-Alkyl, $C_1$-$C_4$-Halogenalkyl, $C_1$-$C_2$-Alkoxy-$C_1$-$C_2$-alkyl, $C_1$-$C_4$-Alkoxy, $C_1$-$C_4$-Halogenalkoxy, $C_1$-$C_4$-Alkylthio, $C_1$-$C_4$-Halogenalkylthio, $C_1$-$C_4$-Alkylamino, Dimethylamino oder Diethylamino steht,

$A^3$ für Wasserstoff, Fluor, Chlor, Brom, $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio, $C_1$-$C_4$-Alkylamino, Dimethylamino oder Diethylamino steht, und

Q für Stickstoff oder eine CH-Gruppierung steht,

sowie deren Hydrogen-chloride, -bromide, und -iodide.

Besonders bevorzugte Verbindungen der Formel (IIa) sind diejenigen, bei denen

$A^1$ für Methyl, Ethyl, Carboxymethyl, Methoxycarbonylmethyl, Ethoxycarbonylmethyl oder Benzyl steht,

$A^2$ für Chlor, Methyl, Ethyl, Trifluormethyl, Methoxy, Ethoxy, Difluormethoxy, Methylthio, Ethylthio, Methylamino, Ethylamino, Dimethylamino oder Diethylamino steht,

$A^3$ für Methyl, Ethyl, Trifluormethyl, Methoxy, Ethoxy, Difluormethoxy, Methylthio, Ethylthio, Methylamino, Ethylamino, Dimethylamino oder Diethylamino steht, und

Q für Stickstoff oder eine CH-Gruppierung steht,

sowie deren Hydrogen-chloride, -bromide und -iodide.

Man erhält die neuen Verbindungen der Formel (IIa), wenn man Thioharnstoffe der Formel (IV)

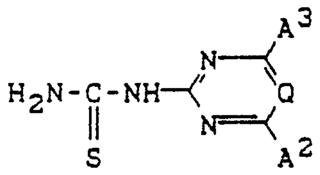

(IV)

in welcher

$A^2$, $A^3$ und Q die oben angegebenen Bedeutungen haben,

mit Alkylierungsmitteln der Formel (V)

$A^1 - X^1$ (V)

in welcher

$A^1$ die oben angegebenen Bedeutungen hat und

$X^1$ für Halogen steht,

gegebenenfalls in Gegenwart eines Säureakzeptors, wie z. B. Kaliumcarbonat, und gegebenenfalls in Gegenwart eines Verdünnungsmittels, wie z. B. Methanol, Ethanol, Isopropanol, Aceton oder Acetonitril, bei Temperaturen zwischen 0 °C und 100 °C umsetzt.

Die als Zwischenprodukte benötigten Thioharnstoffe sind durch die Formel (IV) allgemein definiert. In Formel (IV) haben $A^2$, $A^3$ und Q vorzugsweise bzw. insbesondere diejenigen Bedeutungen, die bereits oben im Zusammenhang mit der Beschreibung der erfindungsgemäßen Verbindungen der Formel (IIa) vorzugsweise bzw. als besonders bevorzugt für $A^2$, $A^3$ und Q angegeben wurden.

Die Thioharnstoffe der Formel (IV) sind bekannt und/oder können nach bekannten Verfahren hergestellt werden (vgl. EP-A 173 313).

Die weiter als Zwischenprodukte benötigten Alkylierungsmittel sind durch die Formel (V) allgemein definiert. In Formel (V) hat $A^1$ vorzugsweise bzw. insbesondere diejenige Bedeutung, die bereits oben im Zusammenhang mit der Beschreibung der erf indungsgemäßen Verbindungen der Formel (IIa) vorzugsweise bzw als besonders bevorzugt angegeben wurde und $X^1$ steht vorzugsweise für Chlor, Brom oder Iod.

Die Zwischenprodukte der Formel (V) sind bekannte organische Synthesechemikalien.

Man erhält die Isothioharnstoffe der Formel (II) beispielsweise auch, wenn man Iminodithiokohlensäurediester der allgemeinen Formel (VI)

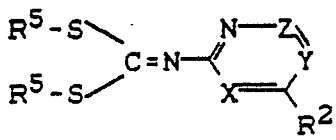

(VI)

in welcher

$R^5$, $R^2$, X, Y und Z die oben angegebenen Bedeutungen haben,

mit Ammoniak, gegebenenfalls in Gegenwart eines Verdünnungsmittels, wie z. B. Methanol, Ethanol, Propanol, Isopropanol, Tetrahydrofuran, Dioxan oder Dimethylformamid, bei Temperaturen zwischen 0 °C und 100 °C umsetzt.

Die als Zwischenprodukte benötigten Iminodithiokohlensäurediester sind Gegenstand einer nicht vorveröffentlichten Patentanmeldung [P 3 829 469.9 (Le A 26 358) vom 31.08.1988].

Man erhält diese Zwischenprodukte der Formel (VI), wenn man Aminoverbindungen der allgmeinen Formel (VII)

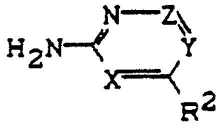

(VII)

in welcher

$R^2$, X, Y und Z die oben angegebenen Bedeutungen haben,

- oder Metallsalze von Verbindungen der Formel (VII) -

mit Schwefelkohlenstoff (Carbondisulfid) in Gegenwart einer starken Base, wie z. B. Natrium- oder Kaliumhydroxid, und in Gegenwart eines Verdünnungsmittels, wie z. B. Dimethylformamid und/oder Wasser, bei Temperaturen zwischen -20 °C und +50 °C umsetzt und in situ weiter mit Alkylierungsmitteln der

allgemeinen Formel (VIII)

$R^5 - X^1$ (VIII)

in welcher

$R^5$ die oben angegebene Bedeutung hat und

$X^1$ für Chlor, Brom oder Iod steht,

bei Temperaturen zwischen 0 °C und 100 °C umsetzt Die nach Verdünnen mit Wasser kristallin anfallenden Produkte der Formel (VI) können durch Absaugen isoliert werden.

Die Aminoverbindungen der Formel (VII) sind bekannt und/oder können nach an sich bekannten Verfahren hergestellt werden (vgl. Chem. Pharm. Bull. 11 (1963), 1382; US-P 4 299 960; EP-A 121 082; EP-A 125 205; EP-A 126 711; EP-A 152 378; EP-A 158 594).

Die Alkylierungsmittel der Formel (VIII) sind bekannte organische Synthesechemikalien.

Die beim erfindungsgemäßen Verfahren zur Herstellung der Oxyguanidine der Formel (I) weiter als Ausgangsstoffe zu verwendenden Hydroxylaminderivate sind durch die Formel (III) allgemein definiert. In Formel (III) steht vorzugsweise

$R^1$ für $C_1$-$C_{12}$-Alkyl [welches gegebenenfalls durch Fluor, Chlor, $C_1$-$C_4$-Alkoxy, $C_1$-$C_4$-Alkylthio, $C_1$-$C_4$-Alkylsulfinyl, $C_1$-$C_4$-Alkylsulfonyl, Carboxy, Cyano oder Nitro substituiert ist], $C_3$-$C_6$-Alkenyl [welches gegebenenfalls durch Fluor oder Chlor substituiert ist], $C_3$-$C_6$-Alkinyl, $C_3$-$C_6$-Cycloalkyl, $C_3$-$C_6$-Cycloalkyl-$C_1$-$C_2$-alkyl, $C_1$-$C_4$-Alkoxy-carbonyl-$C_1$-$C_2$-alkyl, Aminocarbonyl-$C_1$-$C_2$-alkyl, $C_1$-$C_4$-Alkyl-amino-carbonyl-$C_1$-$C_2$-alkyl, Di-($C_1$-$C_4$-alkyl)amino-carbonyl-$C_1$-$C_2$-alkyl, für Phenyl, Phenylethyl, Benzhydryl oder Benzyl [welche gegebenenfalls durch Fluor, Chlor, Brom, Cyano, Nitro, $C_1$-$C_4$-Alkyl, $C_1$-$C_4$-Alkoxy, Carboxy oder $C_1$-$C_4$-Alkoxycarbonyl substituiert sind].

Als Ausgangsstoffe besonders bevorzugt werden die Verbindungen der Formel (III), in welcher

$R^1$ für $C_1$-$C_5$-Alkyl, $C_3$-$C_5$-Alkenyl, $C_1$-$C_3$-Alkoxy-carbonyl-$C_1$-$C_2$-alkyl oder Benzyl (welches gegebenenfalls durch Fluor, Chlor, Methyl, Ethyl, Methoxy, Ethoxy, Methoxycarbonyl und/oder Ethoxycarbonyl substituiert ist).

Als Beispiele für die Ausgangsstoffe der Formel (III) seien genannt:

O-Methyl-hydroxylamin, O-Ethyl-hydroxylamin, O-Propylhydroxylamin, O-Isopropyl-hydroxylamin, O-Butyl-hydroxylamin, O-Isobutyl-hydroxylamin, O-Pentyl-hydroxylamin, O-Allyl-hydroxylamin, O-Methoxycarbonylmethylhydroxylamin, O-Ethoxycarbonylmethyl-hydroxylamin, O-Benzyl-hydroxylamin, O-(4-Chlor-benzyl)-hydroxylamin, O-(4-Fluor-benzyl)-hydroxylamin, O-(4-Methyl-benzyl)hydroxylamin, O-(4-Methoxy-carbonyl-benzyl)-hydroxylamin und O-(4-Ethoxycarbonyl-benzyl)-hydroxylamin.

Die Hydroxylaminderivate der Formel (III) sind bekannt und/oder können nach an sich bekannten Verfahren hergestellt werden (vgl. Chem. Pharm. Bull. 15 (1967), 345; Synthesis 1976, 682; J. Chem. Soc. 1930, 228; Helv. Chim. Acta 45 (1962), 1387).

Das erfindungsgemäße Verfahren zur Herstellung der Oxyguanidine der Formel (1) wird vorzugsweise unter Verwendung von Verdünnungsmitteln durchgeführt. Als Verdünnungsmittel kommen dabei praktisch alle inerten organischen Lösungsmittel in Frage. Hierzu gehören vorzugsweise aliphatische und aromatische, gegebenenfalls halogenierte Kohlenwasserstoffe wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Glykoldimethylether und Diglykoldimethylether, Tetrahydrofuran und Dioxan, Alkohole, wie Methanol, Ethanol, Propanol, Isopropanol, Butanol, Isobutanol, sec-Butanol und tert-Butanol, Ketone wie Aceton, Methyl-ethyl-, Methyl-isopropyl- und Methyl-isobutyl-keton, Ester wie Essigsäuremethylester und -ethylester, Nitrile wie z. B. Acetonitril und Propionitril, Amide wie z. B. Dimethylformamid, Dimethylacetamid und N-Methyl-pyrrolidon sowie Dimethylsulfoxid, Tetramethylensulfon und Hexamethylphosphorsäuretriamid.

Das erfindungsgemäße Verfahren wird im allgemeinen bei Temperaturen zwischen 0 °C und 150 °C, vorzugsweise zwischen 20 °C und 100 °C, und im allgemeinen bei Normaldruck durchgeführt.

Zur Durchführung des erfindungsgemäßen Verfahrens setzt man je Mol Isothioharnstoff der Formel (II) im allgemeinen zwischen 1 und 5 Mol, vorzugsweise zwischen 1,1 und 2,5 Mol, Hydroxylaminderivat der Formel (III) ein.

Die Ausgangsstoffe können in beliebiger Reihenfolge zusammengegeben werden. Das Reaktionsgemisch wird bei der jeweils erforderlichen Temperatur bis zum Ende der Umsetzung gerührt.

Die Aufarbeitung kann nach üblichen Methoden durchgeführt werden. Im allgemeinen wird eingeengt, der Rückstand mit einem geeigneten Losungsmittel, wie z. B. Diethylether, verrieben und das kristallin angefallene Produkt durch Absaugen isoliert.

Die nach dem erfindungsgemäßen Verfahren herzustellenden Oxyguanidine der Formel (I) können als Zwischenprodukte zur Herstellung von Herbiziden verwendet werden (vgl. EP-A 121 082).

Herstellungsbeispiele

Beispiel 1

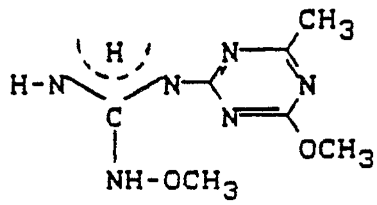

Eine Mischung aus 12,0 g (0,05 Mol) N-(4-Methoxy-6-methyl-s-triazin-2-yl)-S-methyl-isothioharnstoff, 4,70 g (0,10 Mol) O-Methyl-hydroxylamin und 100 ml Ethanol wird 5 Stunden bei 70 °C gerührt. Dann wird eingeengt, der Rückstand mit Diethylether verrieben und das hierbei kristallin angefallene Produkt durch Absaugen isoliert.

Man erhält 8,4g (79 % der Theorie) N′-(4-Methoxy-6-methyl-s-triazin-2-yl )-N″-methoxy-guanidin vom Schmelzpunkt 142 °C.

Analog Beispiel 1 wurden erhalten:

Beispiel 2

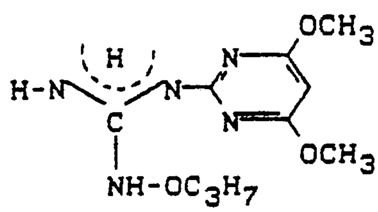

N′-(4,6-Dimethoxy-pyrimidin-2-yl)-N″-propoxy-guanidin
(Ausbeute: 68 % der Theorie).

Beispiel 3

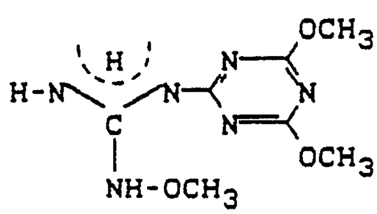

N′-(4,6-Dimethoxy-s-triazin-2-yl )-N″-methoxy-guanidin.

Ausgangsstoffe der Formel (II)

Beispiel (II-1)

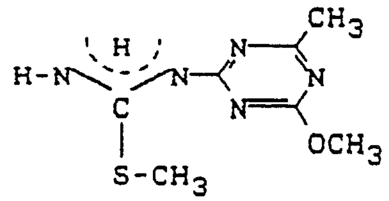

In eine Lösung von 20,0 g (0,08 Mol) N-(4-Methoxy-6-methyl-s-triazin-2-yl)-iminodithiokohlensäure-S,S-dimethylester in 300 ml Ethanol wird bei Temperaturen zwischen 25 °C und 40 °C so lange Ammoniak eingeleitet, bis sich durch Dünnschicht-Chromatographie kein Ausgangsstoff mehr nachweisen läßt. Dann wird das Lösungsmittel im Wasserstrahlvakuum sorgfältig abdestilliert.

Man erhält 17,0g (99 % der Theorie) N-(4-Methoxy-6-methyl-s-triazin-2-yl)-S-methylisothioharnstoff als kristallinen Rückstand vom Schmelzpunkt 112 °C.

Beispiel (II-2)

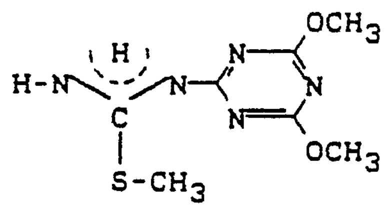

N-(4,6-Dimethoxy-s-triazin-2-yl)-S-methylisothioharnstoff der obigen Formel wird analog Beispiel (II-1) in ebenso guter Ausbeute erhalten.
Schmelzpunkt 109 °C.

Beispiel (II-3)

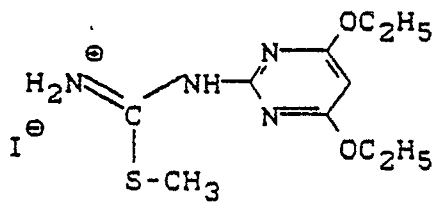

Eine Mischung aus 22,5 g (0,093 Mol) N-(4,6-Diethoxypyrimidin-2-yl)-thioharnstoff, 27,6 g (0,19 Mol) Methyliodid und 130 ml Methanol wird 2 Stunden unter Rückfluß zum Sieden erhitzt.

Anschließend wird eingeengt, der Rückstand mit Diethylether verrieben und das kristallin angefallene Produkt durch Absaugen isoliert.

Man erhält 34,2 g (96 % der Theorie) N-(4,6-Diethoxypyrimidin-2-yl)-S-methylisothioharnstoff-Hydroiodid vom Schmelzpunkt 148 °C.

Analog Beispiel (II-3) können die nachstehend aufgeführten Verbindungen der Formel (II) bzw. (IIa) hergestellt werden:

Beispiel (II-4)

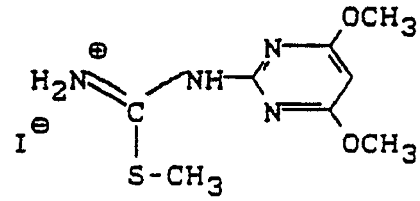

Schmelzpunkt: 137 °C.

Beispiel (II-5)

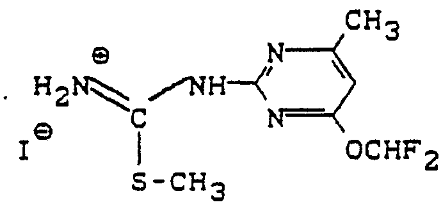

Schmelzpunkt: 161 °C.

Beispiel (II-6)

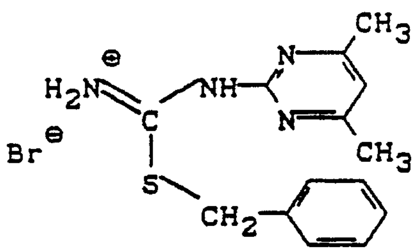

Schmelzpunkt: 147 °C.

Beispiel (II-7)

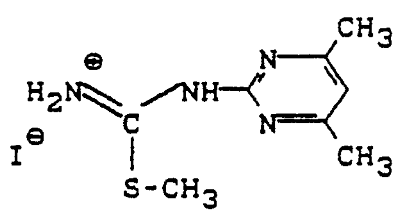

Schmelzpunkt: 243 °C.

Beispiel (II-8)

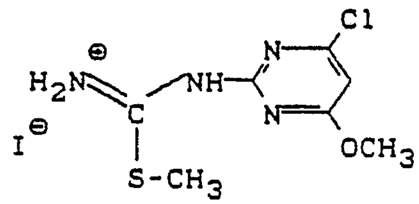

Schmelzpunkt: 152 °C.

Ausgangsstoffe der Formel (VI)

Beispiel (VI-1)

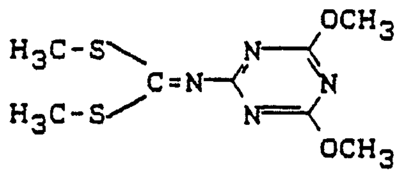

12,5 g (0,22 Mol) Kaliumhydroxidpulver werden portionsweise zu einer Mischung aus 15,4 g (0,10 Mol) 2-Amino-4,6-dimethoxy-s-triazin, 8,5 g (0,11 Mol) Schwefelkohlenstoff und 80 ml Dimethylformamid gegeben, wobei die Innentemperatur unter 35 °C gehalten wird. Das Reaktionsgemisch wird dann 30 Minuten bei 45 °C gerührt. Anschließend werden 31 g (0,22 Mol) Methyliodid unter weiterem Rühren tropfenweise dazu gegeben und das Rühren wird noch 60 Minuten bei 40 °C fortgesetzt. Nach Abkühlen wird mit 250 ml Wasser verdünnt und das hierbei kristallin angefallene Produkt durch Absaugen isoliert.

Man erhält 23,6 g (90 % der Theorie) N-(4,6-Dimethoxy-s-triazin-2-yl)-iminodithiokohlensäure-S,S-dimethylester vom Schmelzpunkt 123 °C.

Beispiel (VI-2)

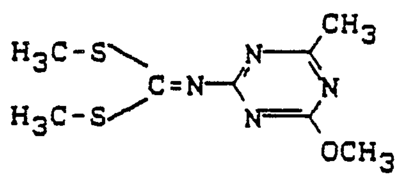

N-(4-Methoxy-6-methyl-s-triazin-2-yl)-iminodithiokohlensäure-S,S-dimethylester der obigen Formel wird analog Beispiel (VI-1) in ebenso guter Ausbeute erhalten Schmelzpunkt: 78 °C.

**Ansprüche**

1. Verfahren zur Herstellung von Oxyguanidinen der allgemeinen Formel (I)

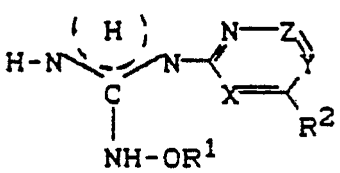

(I)

in welcher
$R^1$ für einen jeweils gegebenenfalls substituierten Rest aus der Reihe Alkyl, Alkenyl, Alkinyl,Cycloalkyl, Cycloalkylalkyl, Aralkyl und Aryl steht,
$R^2$ für Wasserstoff, Halogen, Hydroxy, Amino oder einen gegebenenfalls substituierten Rest aus der Reihe Alkyl, Alkoxy, Alkylthio, Alkylamino und Dialkylamino steht,
X für Stickstoff oder eine CH-Gruppierung steht,
Y für Stickstoff oder eine $CR^3$-Gruppierung steht, worin
$R^3$ für Wasserstoff, Halogen, Cyano, Alkyl, Formyl, Alkylcarbonyl oder Alkoxycarbonyl steht, und
Z für Stickstoff oder eine $CR^4$-Gruppierung steht, worin
$R^4$ für Wasserstoff, Halogen, Hydroxy, Amino oder einen gegebenenfalls substituierten Rest aus der Reihe Alkyl, Alkoxy, Alkylthio, Alkylamino und Dialkylamino steht,
dadurch gekennzeichnet, daß man Isothioharnstoffe der allgemeinen Formel (II)

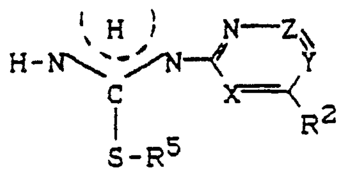

(II)

in welcher
$R^2$, X, Y und Z die oben angegebenen Bedeutungen haben und
$R^5$ für jeweils gegebenenfalls substituiertes Alkyl oder Aralkyl steht,
- oder deren Hydrogenhalogenide -
mit Hydroxylaminderivaten der allgemeinen Formel (III)
$H_2N - O - R^1$ (III)
in welcher
$R^1$ die oben angegebene Bedeutung hat,
gegebenenfalls in Gegenwart eines Verdünnungsmittel bei Temperaturen zwischen 0°C und 150°C umsetzt.

2. Verfahren nach Anspruch 1, dadurch gekennzeichnet, daß man bei Temperaturen zwischen 20°C und 100°C arbeitet.

3. Verfahren nach Anspruch 1, dadurch gekennzeichnet. daß man je Mol Isothioharnstoff der Formel (II) 1 - 5 Mol, vorzugsweise 1,1 - 2,5 Mol, Hydroxylaminderivat der Formel (III) einsetzt.

4. Isothioharnstoffe der Formel (IIa)

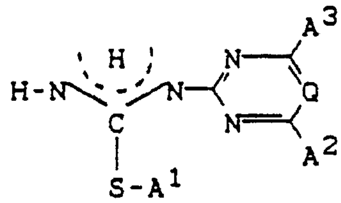

(IIa)

in welcher
$A^1$ für jeweils gegebenenfalls substituiertes Alkyl oder Aralkyl steht,
$A^2$ für Halogen oder einen gegebenenfalls substituierten Rest aus der Reihe Alkyl, Alkoxy, Alkylthio, Alkylamino und Dialkylamino steht,
$A^3$ für Wasserstoff, Halogen oder einen gegebenenfalls substituierten Rest aus der Reihe Alkyl, Alkoxy, Alkylthio, Alkylamino und Dialkylamino steht, und
Q für Stickstoff oder eine CH-Gruppierung steht,
sowie deren Hydrogenhalogenide, ausgenommen die Verbindung N-(4,6-Dimethoxy-s-triazin-2-yl)-S-methyl-isothioharnstoff.

5. Isothioharnstoffe der Formel (II a) gemäß Anspruch 4, dadurch gekennzeichnet, daß darin
$A^1$ für $C_1$-$C_4$-Alkyl (welches gegebenenfalls durch Cyano, Carboxy oder $C_1$-$C_4$-Alkoxy-carbonyl substituiert ist) oder für Benzyl (welches gegebenenfalls durch Halogen und/oder $C_1$-$C_2$-Alkyl substituiert ist) steht,
$A^2$ für Fluor, Chlor, Brom, $C_1$-$C_4$-Alkyl, $C_1$-$C_4$-Halogenalkyl, $C_1$-$C_2$-Alkoxy-$C_1$-$C_2$-alkyl, $C_1$-$C_4$-Alkoxy, $C_1$-$C_4$-Halogenalkoxy, $C_1$-$C_4$-Alkylthio, $C_1$-$C_4$-Halogenalkylthio, $C_1$-$C_4$-Alkylamino, Dimethylamino oder Diethylami-

no steht,
$A^3$ für Wasserstoff, Fluor, Chlor, Brom, $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio, $C_1$-$C_4$-Alkylamino, Dimethylamino oder Diethylamino steht, und
Q für Stickstoff oder eine CH-Gruppierung steht,
sowie deren Hydrogen-chloride, -bromide, und -iodide, ausgenommen die Verbindung H-(4,6-Dimethoxy-s-triazin-2-yl)-S-methylisothioharnstoff.

6. Isothioharnstoffe der Formel (II a) gemäß Anspruch 4, dadurch gekennzeichnet, daß darin
$A^1$ für Methyl, Ethyl, Carboxymethyl, Methoxycarbonylmethyl, Ethoxycarbonylmethyl oder Benzyl steht,
$A^2$ für Chlor, Methyl, Ethyl, Trifluormethyl, Methoxy, Ethoxy, Difluormethoxy, Methylthio, Ethylthio, Methylamino, Ethylamino, Dimethylamino oder Diethylamino steht,
$A^3$ für Methyl, Ethyl, Trifluormethyl, Methoxy, Ethoxy, Difluormethoxy, Methylthio, Ethylthio, Methylamino, Ethylamino, Dimethylamino oder Diethylamino steht, und
Q für Stickstoff oder eine CH-Gruppierung steht,
sowie deren Hydrogen-chloride, -bromide und -iodide, ausgenommen die Verbindung N-(4,6-Dimethoxys-triazin-2-yl)-S-methyl-isothioharnstoff.

7. Verfahren zur Herstellung von Isothioharnstoffen der Formel (II a) gemäß Anspruch 4, dadurch gekennzeichnet, daß man Thioharnstoffe der Formel (IV)

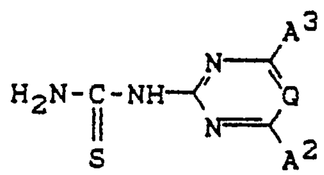

(IV)

in welcher
$A^2$, $A^3$ und Q die in Anspruch 4 angegebenen Bedeutungen haben,
mit Alkylierungsmitteln der Formel (V)
$A^1$ - $X^1$ (V)
in welcher
$A^1$ die in Anspruch 4 angegebenen Bedeutungen hat und
$X^1$ für Halogen steht,
gegebenenfalls in Gegenwart eines Säureakzeptors und gegebenenfalls in Gegenwart eines Verdünnungsmittels bei Temperaturenzwischen 0 °C und 100 °C umsetzt.